Europäisches Patentamt

European Patent Office

Office européen des brevets

(19)

(11) Veröffentlichungsnummer: **0 004 019**
**B1**

(12) **EUROPÄISCHE PATENTSCHRIFT**

(45) Veröffentlichungstag der Patentschrift: **29.07.81**

(21) Anmeldenummer: **79100573.9**

(22) Anmeldetag: **26.02.79**

(51) Int. Cl.³: **C 07 D 301/32,**
**C 07 D 303/04**

(54) **Verfahren zur Reinigung von Epoxiden.**

(30) Priorität: **11.03.78 DE 2810662**

(43) Veröffentlichungstag der Anmeldung:
**19.09.79 Patentblatt 79/19**

(45) Bekanntmachung des Hinweises auf die Patenterteilung:
**29.07.81 Patentblatt 81/30**

(84) Benannte Vertragsstaaten:
**BE DE FR GB IT NL**

(56) Entgegenhaltungen:
**DE - A - 2 047 292**
**DE - C - 681 866**
**US - A - 3 149 131**
**US - A - 3 597 452**

**CHEMICAL ABSTRACTS, Vol. 71,**
**Nr. 17, 27—10—1969, Nr. 81128p**
**Columbus, Ohio, U.S.A.**
**H. OUCHI et al.: "Purification of**
**alkylene oxides", Seite 372, linke**
**Spalte**

(73) Patentinhaber: **BAYER AG**
**Zentralbereich Patente, Marken und Lizenzen**
**D-5090 Leverkusen 1, Bayerwerk (DE)**

(73) Patentinhaber: **Degussa Aktiengesellschaft**
**Degussa AG Fachbereich Patente Rodenbacher**
**Chaussee 4 Postfach 1345**
**D-6450 Hanau 1 (Stadtteil Wolfgang) (DE)**

(72) Erfinder: **Seifert, Hermann, Dr.**
**Ruwergasse 4**
**D-5000 Köln 80 (DE)**
Erfinder: **Waldmann, Helmut, Dr.**
**Carl-Rumpff-Strasse 59**
**D-509 Leverkusen (DE)**
Erfinder: **Wirthwein, Rolf, Dr.**
**Fuerstenbergstrasse 4**
**D-6450 Hanau 9 (DE)**
Erfinder: **Hofen, Willi**
**Suedring 54**
**D-6451 Rodenbach (DE)**

Courier Press, Leamington Spa, England.

EP 0 004 019 B1

## Verfahren zur Reinigung von Epoxiden

Die vorliegende Erfindung betrifft ein verbessertes Verfahren zur Reinigung von Epoxiden oder Epoxide enthaltenden Gemischen. Insbesondere ist die Entfernung von Carbonylgruppen enthaltenden Verbindungen aus Epoxiden oder aus Gemischen, in denen Epoxide vorliegen, Gegenstand der vorliegenden Erfindung. Epoxide, wie Äthylenoxid, Propylenoxid, Butylenoxid, Styroloxid, Epichlorhydrin oder Glycid sind wichtige Zwischenprodukte, die eine breite Anwendung auf dem Gebiet der Kunststoffe, z.B. bei Polyurethanen oder auf dem Klebstoffsektor finden.

Mengenmäßig sind die Epoxide der niederen Olefine, beispielsweise Äthylen- und Propylenoxid, bei weitem wichtigsten monomeren Verbindungen dieser Art. An die Reinheit dieser Epoxide werden im Hinblick auf die Qualität der Endprodukte sehr hohe Anforderungen gestellt. Insbesondere Verbindungen, die Carbonylgruppen enthalten, z.B. die jeweils 1 bis 5 C-Atome enthaltenden Aldehyde, Ketone und Ester, dürfen nur in ganz geringen Konzentrationen in Epoxiden vorhanden sein. So ist es beispielsweise notwendig, die Konzentration an Acetaldehyd im Propylenoxid auf unter 50 ppm zu halten, da bei höheren Acetaldehydgehalten beispielsweise bei der Herstellung von Polyäthern aus den Epoxiden gefärbte Produkte entstehen. Im folgenden wird im wesentlichen am Beispiel des Propylenoxids aufgeführt, wie zahlreich, entsprechend diesen hohen Anforderungen an die Reinheit des Produktes, die Vorschläge sind, die gemacht wurden, um die genannten Verbindungen zu entfernen.

Alle bisher bekannten Herstellungsverfahren für niedermolekulare Epoxide basieren auf der Oxidation des entsprechenden Olefins. Als Oxidationsmittel werden dabei Chlor in Verbindung mit Alkali, molekularer Sauerstoff, organische Hydroperoxide oder Percarbonsäuren verwendet. Auch elektrochemische Verfahren sind bekannt geworden. Bei all diesen Verfahren entstehen mehr oder weniger große Mengen an Nebenprodukten, die Sauerstoff in Form von Carbonylgruppen enthalten. Im Falle der Herstellung von Propylenoxid werden z.B. Formaldehyd, Acetaldehyd, Aceton, Propionaldehyd und Ester niedermolekularer Carbonsäuren, beispielsweise Methylformiat, als Nebenprodukte beobachtet. Diese störenden Nebenprodukte entstehen nicht nur durch den oxidativen Abbau des Propylens, sondern können auch aus Propylenoxid während der Reaktion oder bei der Aufarbeitung der Reaktionsgemische gebildet werden. Zur Entfernung der Carbonylgruppen enthaltenden Nebenprodukte aus dem Propylenoxid sind bereits eine Reihe von Reinigungsverfahren vorgeschlagen worden, die entweder auf einer physikalisch-chemischen Trennoperation oder auf einer chemischen Umsetzung beruhen.

Als physikalisch-chemische Trennoperation wendet man zur Reinigung von Epoxiden die Destillation an. Dabei erbringen meist jedoch nur die Extraktivdestillationsverfahren hinreichend befriedigende Ergebnisse, d.h. Verfahren, bei denen unter Zuhilfenahme eines zusätzlichen Lösungsmittels im Gegenstrom gearbeitet wird.

Für die Abtrennung von Methylformiat aus Propylenoxid wird beispielsweise gemäß der DT—PS 1 224 293 vorgeschlagen, eine Extraktivdestillation mit Kohlenwasserstoffen durchzuführen. Kohlenwasserstoffe, wie Olefine, Aromaten und deren Mischungen, werden in der US—PS 3 337 425 als geeignete Extraktionsmittel beschrieben, um Sauerstoff enthaltende Verunreinigungen, die innerhalb eines Bereiches von plus oder minus 5°C vom Siedepunkt des Epoxids sieden, aus diesem durch extraktive Destillation abzutrennen. Als aus Propylenoxid abzutrennende Verbindungen werden Methylformiat und Acetaldehyd genannt.

Eine wäßrige, alkalische Lösung wird als Extraktionsmittel in der US—PS 2 622 060 empfohlen, um mittels einer Extraktivdestillation Verunreinigungen, wie Acetaldehyd und Methylformiat, aus Propylenoxid entfernen zu können. Diese Arbeitsweise in Gegenwart von wäßrigem Alkali verursacht hohe Verluste an Epoxid, da Epoxide in Gegenwart von wäßrigem Alkali bekanntermaßen zu Glykolen verseifen. Darüberhinaus ist die Abtrennung von Wasser aus Propylenoxid nur mit sehr großem Destillationsaufwand möglich (s. DT—OS 2 015 602). Alle Extraktiv-Destillations-Verfahren zur Abtrennung von Carbonylverbindungen aus Propylenoxid sind, abgesehen von den Nachteilen, die durch die Verluste an Epoxid entstehen können, aufwendig, da zumindest eine weitere Destillationseinheit erforderlich ist, um das Extraktionsmittel zu reinigen und möglichst verlustfrei im Kreis führen zu können. Ein weiterer, nicht unerheblicher Nachteil ist darin zu sehen, daß das verwendete Extraktionsmittel des Olefinoxid verunreinigt.

Die einfache destillative Abtrennung der erwähnten Carbonylgruppen enthalten Verunreinigungen aus Propylenoxid ist nicht mit hinreichend befriedigendem Ergebnis möglich, obwohl die beträchtlichen Unterschiede der Siedepunkte dieser Verbindungen diese eigentlich hätten erwarten lassen müssen.

Die beobachteten Schwierigkeiten hängen teilweise mit der Ausbildung von in der Nähe des Propylenoxids siedenden azeotropen Gemischen zusammen. Als besonders schwierig ist die Abtrennung der niederen Aldehyde anzusehen. In der DT—OS 2 454 115 (S. 2, 3. Absatz) heißt es hierzu, daß die Abtrennung von Aldehyden — die im allgemeinen in Mengen von bis zu etwa 2 Gew.-% zugegen sind — ein Problem darstellt, da ein für technische Zwecke befriedigendes Propylenoxid weniger als etwa 100 ppm vorzugsweise weniger als etwa 20 ppm Aldehyde, ausgedrückt als Propionaldehyd, enthalten soll.

Bezüglich der Propylenoxid-Verluste, die bei der einfachen destillativen Trennung von Acetaldehyd und Propylenoxid- also ohne Anwendung eines Extrationsmittels — auftreten, wird in der DT—OS 2 454 115 (Seite 3, 2. Absatz) erwähnt, daß etwa 30% des Propylenoxids in dem Acetaldehyd enthaltenden Destillat verloren gehen, das im Rahmen der Propylenoxidgewinnung erhalten wird.

In der DT—OS 2 454 115 wird vorgeschlagen, um diese Verluste zu verringern, die Abtrennung der Aldehyde Acetaldehyd und Propionaldehyd getrennt in zwei Destillationseinheiten vorzunehmen, wobei zunächst Acetaldehyd als Destillat erhalten wird. Neben dem großen Aufwend, hervorgerufen durch die Verwendung von Kolonnen mit 90 bzw. 62 Böden und hohen Rücklaufverhältnissen, ist in der zweimaligen Temperaturbelastung, der das Propylenoxid in Gegenwart nicht unerheblicher Mengen Wasser ausgesetzt ist, ein weiterer bedeutender Nachteil zu sehen.

Andere bisher bekanntgewordene Verfahren zur Entfernung von Carbonylgruppen enthaltenden Verunreinigungen aus Epoxiden setzen chemische Hilfsmittel ein, um durch Bindung von Carbonylverbindungen die Epoxide zu reinigen. Sie haben jedoch so erhebliche Nachteile, daß eine Anwendung für technische Verfahren durchweg ausscheidet.

In der US—PS 3 816 478 wird vorgeschlagen, Formaldehyd, Acetaldehyd oder Propionaldehyd durch Behandlung mit festem Natriumbisulfit aus dem Propylenoxid zu entfernen. Zur Durchführung dieses Verfahrens wird das Natriumbisulfit in einem Festbett angeordnet und das verunreinigte Propylenoxid über dieses Bett geleitet, wobei sich aus dem Sulfit und den Aldehyden Additionsverbindungen ausbilden. Das zur Reinigung gelangende organische Einsatzprodukt muß etwa 2 Gew.-% Wasser enthalten. Nachteile des Verfahrens sind vor allem in der als Nebenreaktion in erheblichem Maße ablaufenden Umwandlungen des Epoxids zu sehen. Daneben wird das zu reinigende Epoxid mit Schwefeldioxid verunreinigt. Wenn die Oberfläche des Bisulfitgranulats mit der Aldehyd-Additions-Verbindung belegt ist, muß das Festbett durch Spülen mit einem inerten, organischen Lösungsmittel gereinigt und danach bei höherer Temperatur mit Luft oder Stickstoff behandelt werden. Diese beiden Verfahrensschritte belasten, abgesehen von der bei kontinuierlicher Arbeitsweise vorliegenden Notwendigkeit einen zweiten Waschturm zu errichten, das Verfahren erheblich und lassen dessen Durchführung wenig vorteilhaft erscheinen.

Ein weiteres Verfahren zur Entfernung von Carbonylgruppen enthaltenden Verbindungen aus Propylenoxid wird in der GB—PS 1 035 866 vorgeschlagen. Die chemische Veränderung der Verunreinigungen wird mittels Natriumborhydrid vorgenommen. Dieses besitzt den Vorteil, selektiv mit den Carbonylgruppen enthaltenden Verbindungen zu reagieren, hat jedoch auch schwerwiegende Nachteile. So werden bei der Umsetzung von Natriumborhydrid mit Aldehyden, Ketonen und Estern borhaltige organische Verbindungen gebildet, die weitgehend unlöslich sind und bei der technischen Durchführung des Verfahrens aufwendige Filtereinrichtungen notwendig machen. Natriumborhydrid selbst ist wiederum in geringem Maße im Propylenoxid löslich, was zu Belägen und Verstopfungen in Rohren und Wärmeaustauschern bei der nachfolgenden Destillation führt.

Eine weitere Methode zur Reinigung von Propylenoxid mit chemischen Mitteln ist in der jap. — AS 9650/69 (Chemical Abstracts 71, 81 128 p (1969)) beschrieben. Zur Entfernung von Propionaldehyd aus Proylenoxid wird die Verwendung von wäßrigen Lösungen von Hydrazin vorgeschlagen. Ein Nachteil dieses Verfahrens besteht darin, daß Reaktionszeiten von zwanzig Stunden bei Raumtemperatur notwendig sind, um die Umsetzung des Propionaldehyds quantitativ zu gestalten. Reaktionszeiten dieser Größenordnung sind technisch nur mit erheblichem Aufwand zu realisieren, da bei diesen Verweilzeiten sehr große Reaktorvolumina zur Verfügung stehen müssen. Daneben treten als Folge dieser langen reaktionszeiten auch Verluste an Propylenoxid auf, die hervorgerufen werden durch die ebenfalls erfolgende, nunmehr nicht unerhebliche Nebenreaktion des Propylenoxids mit dem Hydrazin oder mit dem Wasser der Hydrazinlösung. Ein weiterer Nachteil des Verfahrens gemäß dieser jap. — AS 9650/69 ist in der Tatsache zu sehen, daß bei Anwendung dieses Verfahrens auf die Entfernung von Acetaldehyd aus Propylenoxid Produkte entstehen, die nur eine sehr geringe Löslichkeit in dem zu reinigenden Epoxid besitzen, so daß bei der technischen Durchführung aufwendige Vorkehrungen getroffen werden müssen, damit keine Ablagerungen und Verstopfungen, bedingt durch diese unlöslichen Produkte, in Verdampfern, Apparaten und Rohrleitungen auftreten.

Das Verfahren gemäß der jap. — AS 9650/69 ist also mit den oben geschilderten Nachteilen und Einschränkungen nur für die Entfernung von Propionaldehyd geeignet. Ist beispielsweise aus einem Propylenoxid enthaltenden Gemisch Acetaldehyd als einzige im Gemisch vorliegende Carbonylverbindung zu entfernen, so wird es zur Umgehung der durch das Auftreten von unlöslichen Produkten bedingten Schwierigkeiten notwendig, dem Gemisch zusätzlich zum bereits vorhandenen Acetaldehyd auch noch Propionaldehyde — gleichsam als Lösungsvermittler — zuzusetzen. Der Zusatz von Propionaldehyd bedeutet aber gleichzeitig auch einen höheren Verbrauch an wäßriger Hydrazinlösung.

Allen dieser Verfahren, die zur Entfernung von Carbonylgruppen enthaltenden Verunreinigungen aus Epoxiden eine chemische Reaktion benutzen, ist der Nachteil gemeinsam, daß das benötigte, im Überschuß eingesetzte Reagenz ebenso wie die aus dem Reagenz und den Carbonylgruppen enthaltenden Verbindungen entstehenden Reaktionsprodukte zusätzliche Verunreinigungen darstellen. Neben dem zwangsweise notwendigen Reaktionsschritt ist somit eine nachfolgende Destillativtrennung des Reaktionsgemisches unumgänglich. Diese Destillation wird meist noch durch die Bildung komplizierter zusammengesetzter, mit dem Epoxid azeotrop seidender Gemisch erschwert, wenn nicht gar unmöglich gemacht.

Zusammenfassend ist also über die bisher bekanntgewordenen Verfahren zur Reinigung von Epoxiden, insbesondere von Propylenoxid, von Carbonylverbindungen festzustellen, daß keines dieser Verfahren eine zufriedenstellende Lösung dieses Problems bietet.

Demgegenüber wurde nun ein technisch einfaches und wirtschaftlich vorteilhaftes Verfahren zur Reinigung von Epoxiden gefunden, das dadurch gekennzeichnet ist, daß man das Epoxid oder ein Epoxid enthaltendes Gemisch, das bezogen auf das Epoxid, bis zu 2 Gew.-% 1 bis 5 C-Atome enthaltende Carbonylverbindungen enthält, in den mittleren Bereich einer Destillationseinheit einleitet, oberhalb des Zulaufs des Epoxid enthaltenden Produktstroms eine Verbindung, die eine oder mehrere unsubstituierte $NH_2$-Gruppen aufweist, einleitet und oberhalb der Einleitungsstelle dieser mindestens ein $HN_2$-Gruppe enthaltenden Verbindung in die Destillationskolonne das gereinigte Epoxid als Kopfprodukt oder in einem Seitenstrom abzieht.

Man kann dieses Verfahren beispielsweise durchführen, indem man eine Destillationseinheit verwendet, die einen Abtriebsteil mit 1 bis 50 theoretischen Trennstufen, einen Aufkonzentrierteil mit 1 bis 70 theoretischen Trennstufen, eine Verdampfereinheit und eine Kondensationseinrichtung enthält, wobei man zwischen dem Abtriebsteil und dem Aufkonzentrierteil das Epoxid oder das Epoxid enthaltende Gemisch und 1 bis 20 theoretische Trennstufen oberhalb davon die Verbindung mit der unsubstituierten $NH_2$-Gruppe einleitet und 1 bis 50 theoretische Trennstufen oberhalb der Eingabestelle der die unsubstituierte Aminogruppe enthaltenden Verbindung das gereinigte Epoxid als Kopfprodukt oder in einem Seitenstrom abzieht. Das erfindungsgemäße Verfahren kann vorteilhafterweise so durchgeführt werden, daß man beispielsweise Äthylenoxid, Propylenoxid, 1,2-Epoxybutan, 2,3-Epoxybutan, Vinyloxiran oder Styroloxid oder ein Gemisch, das eines dieser Epoxide enthält, wobei das Epoxid oder das Epoxid enthaltende Gemisch, bezogen auf das Epoxid, bis zu 2 Gew.-% 1 bis 5 C-Atome enthaltende Carbonylverbindungen enthält, einer Destillationseinheit zuführt, die aus einem Abtriebsteil mit 1 bis 20 theoretischen Trennstufen, einem Aufkonzentrierteil einer Verdampfereinheit und einer Kondensationsvorrichtung besteht, wobei man 1 bis 10 theoretische Stufen oberhalb des Zulaufs des Epoxids oder des Epoxid enthaltenden Gemisches die Verbindung mit der unsubstituierten $NH_2$-Gruppe einleitet und bei einem Kopfdruck von 0,01 bis 2,5 bar und einem Verhältnis von Rücklauf auf die Kolonne zu am Kopf entnommenem Destillat von 0,1 bis 10:1 das gereinigte Epoxid 5 bis 40 theoretische Stufen oberhalb des Zulaufs der die $NH_2$-Gruppe enthaltenden Verbindung als Kopfprodukt oder in einem Seitenstrom abzieht.

Als Destillationseinheit, die für das erfindungsgemäße Verfahren Verwendung finden kann, kommen übliche Kolonnen, wie Bodenkolonnen oder Füllkörperkolonnen, infrage. Ebenso geeignet sind Kolonnen, die mit Packungen aus Draht- oder Glasfasergewebe oder mit keramischen Packungen ausgerüstet sind. Vorteilhafterweise wird der zwischen den beiden Zulaufstellen befindliche Teil der Kolonne als Bodenkolonne gestaltet.

Als Verdampfer können die bekannten Konstruktionen, wie Dünnschicht-, Fallstrom-, Umlauf- oder Kletterverdampfer eingesetzt werden. Umlaufverdampfer sind im allgemeinen gut geeignet.

Als Materialien für den gesamten Destillationsturm, den Verdampfer und den Kondensator können die im Chemieanlagenbau üblicherweise hierfür verwendeten Werkstoffe eingesetzt werden, z.B. Stahl, Glas, Edelstähle und emaillierte Stähle.

Die Epoxide, die nach dem erfindungsgemäßen Verfahren in vorteilhafter Weise gereinigt werden können, sind Mono- und Polyepoxide mit 2 bis 20 C-Atomen, vorzugsweise mit 2 bis 10 C-Atomen, insbesondere die Mono- und Diepoxide der niederen Olefine, z.B. von Olefinen mit 2—6 C-Atomen, wie Äthylenoxid, Propylenoxid, Butylenoxid (1,2-Epoxibutan, 2,3-Epoxibutan), Isobutylenoxid, Vinyloxiran (Butadienmonoepoxid) oder Epoxide von durch Chlor, Aryl und Hydroxyl substituierten Olefinen, wie Epichlorhydrin, Glycid, 1,2-Dichlor-3,4-epoxibutan, 1,4-Dichlor-2,3-epoxibutan, Styroloxid oder 1,2-Diphenyläthylenoxid. Besonders vorteilhaft läßt sich das erfindungsgemäße Verfahren für die Reinigung von Äthylen- und Propylenoxid, sowie von 1,2-Epoxibutan und 2,3-Epoxibutan einsetzen. In ganz besonders vorteilhafter Weise läßt sich das erfindungsgemäße Verfahren für die Reinigung von Propylenoxid einsetzen.

Der in die Destillationseinheit eingeführte, das Epoxid enthaltende Strom kann überwiegend aus Epoxid bestehen oder eine Lösung des Epoxids sein, wobei, bezogen auf des Epoxid, bis zu 2 Gew.-% Carbonylverbindungen vorhanden sein können. Sofern das eingesetzte Epoxid in Form einer Lösung eingesetzt wird, kommen Produktströme infrage, die bei der Herstellung von Epoxiden nach technischen Verfahren bei der Auftrennung der Reaktionsgemische anfallen. Als Lösungsmittel für das eingesetzte Epoxid kommen also vorzugsweise solche infrage, die bei den Herstellungsverfahren für die Epoxide Verwendung finden. Solche Lösungsmittel sind beispielsweise Äthylbenzol, Methylphenylcarbinol, Benzol, Toluol, Xylol, Essigsäure, tert.-Butylalkohol, Wasser und Dichlorpropan, sowie Gemische dieser Verbindungen. Die das Epoxid verdünnende Menge an Lösungsmittel kann in weiten Grenzen schwanken, beispielsweise von 10 bis 90 Gew.-%, und ist für das erfindungsgemäße Verfahren nicht von besonderer Bedeutung.

Die Carbonylgruppen enthaltenden Verunreinigungen, die üblicherweise die Gewinnung der Epoxide in reiner Form erschweren und mit dem erfindungsgemäßen Verfahren beseitigt werden können, sind beispielsweise Aldehyde, Ketone und Ester, die jeweils 1 bis 5 C-Atome im Molekül enthalten, wie Formaldehyd, Acetaldehyd, Propionaldehyd, Aceton, Acrolein, Diacetyl, Methyläthylketon, Hydroxyaceton, Butyraldehyd, Crotonaldehyd, Methylformiat, Essigsäuremethylester, Essigsäureisopropylester und Propionsäureäthylester. Im allgemeinen sieden diese Verbindungen unterhalb des zur Reinigung gelangenden Epoxids. Aber auch Carbonylverbindungen, die einen höheren Siedepunkt als das Epoxid besitzen, aber mit dem Epoxid und gegebenenfalls Wasser ein tiefer als das Epoxid siedendes oder in der Nähe des Siedepunkts des Epoxids siedendes Azeotrop bilden, können nach dem erfindungsgemäßen Verfahren erfolgreich abgetrennt werden, ebenso wie Carbonylverbindungen, die niedriger als das Epoxid sieden und ein underhalb des Siedepunkts des Epoxids liegendes, teilweise erhebliche Mengen an Epoxid enthaltendes Azeotrop bilden. Ein besonderer Vorteil des erfindungsgemäßen Verfahrens ist es, daß die Epoxide von Gemischen, in denen Aldehyde, Ketone und Ester niederer Carbonsäuren in unterschiedlicher Konzentration mit sehr verschiedener chemischer Struktur gleichzeitig vorliegen, in einfacher Weise abgetrennt werden können. So ist es beispielsweise ohne Schwierigkeiten möglich, Formaldehyd, Acetaldehyd, Acrolein, Propionaldehyd, Aceton und Methylformiat in einem Destillationsschritt von Propylenoxid zu trennen, ohne das dabei nachweisliche Verluste an Propylenoxid auftreten.

In das erfindungsgemäße Verfahren wird die Verbindung, die eine oder mehrere unsubstituierte $NH_2$-Gruppen im Molekül enthält, an der angegebenen Stelle in die Destillationseinheit eingespeist. Dafür kommt praktisch jede chemische Verbindung in Betracht, die in ihrem Molekül mindenstens eine $NH_2$-Gruppe enthält.

Als Verbindungen dieser Art, für die Durchführung des erfindungsgemäßen Verfahrens geeignet sind, kommen beispielsweise im einzelnen in Frage:

Monoamine der allgemeinen Formel $R—NH_2$, wobei R ein substituierter oder unsubstituierter geradkettig oder verzweigt vorliegender aliphatischer oder ein aromatischer Rest ist, wie beispielsweise Äthylamin, n-Propylamin, Isopropylamin, n-Butylamin, Isobutylamin, Äthanolamin oder Anilin, Toluidin, o-Nitroanilin und $\alpha$- oder $\beta$-Naphthylamin. Weiterhin können für das erfindungsgemäß Verfahren verwendet werden: Diamine, bei denen sich zwischen den beiden $NH_2$-Gruppen im Molekül Alkylenreste mit z.B. eins, zwei oder drei C-Atomen oder ein Phenylenrest befinden, wie beispielsweise Äthylendiamin, 1,2-Diaminopropan, 1,3-Diaminopropan oder o-Phenylendiamin; aber auch Aminale, Säureamide von Carbon-, Sulfon- oder Phosphonsäuren wie beispielsweise Acetamid, Propionsäureamid, Isobutyramid, Benzolsulfonamid, p-Toluolsulfonamid, das Amid des Monomethylesters der Methylphosphonsäure oder Amide der Kohlensäure wie Harnstoff oder N-Alkyl- oder N,N-Dialkylharnstoff; ebenso sind geeignet Aminocarbonsäuren wie Glycin, Alanin, Norvalin, Methionin, Valin und Lysin sowie Anthranilsäure oder die Nitrile von $\alpha$-Aminocarbonsäuren wie beispielsweise $\alpha$-Amino-propionitril. Neben Aminen und Amiden sind für das erfindungsgemäße Verfahren auch Cyanamid oder Hydroxylamin sowie Hydrazin und Hydrazinderivate, die eine unsubstituierte $NH_2$-Gruppe enthalten, geeignet. Beispielsweise seien genannt Hydrazin, Hydrazinmonohydrat, Monoalkylhydrazine, wie Methyl-, Äthyl-, propyl-, Butyl- und Isopropylhydrazin, Monoarylhydrazine, wie Phenylhydrazin und 2,4-Dinitrophenylhydrazin, N,N-Dialkylhydrazine, wie N,N-Dimethylhydrazin und Hydrazide, wie Semicarbazid, Acethydrazid, Benzoesäurehydrazid, Isobuttersäurehydrazid und Hydrazide von Thiocarbonsäuren.

Neben den Verbindungen mit den freien $NH_2$-Gruppen im Molekül können auch die entsprechenden Salze von Mineral- und Carbonsäuren eingesetzt werden, wie beispielsweise die Ammoniumsalze der aufgeführten $NH_2$-Gruppen enthaltenden Verbindungen, die sich von Salzsäure, Schwefel- und Essigsäure ableiten. Als Beispiele seien genannt tert.-Butylaminhydrochlorid, das Hydrogensulfat von 1,2-Diaminoäthan oder auch Hydraziniumsalze wie Hydrazinsulfat.

Als besonders geeignet für das erfindungsgemäße Verfahren sind n-Alkylamine mit 2 bis 5 C-Atomen, Amide und Hydrazide von Carbonsaüren mit 1 bis 5 C-Atomen sowie Hydroxylaminhydrochlorid zu nennen.

Ganz besonders geeignet sind 1,2-Diaminoalkane mit 2 bis 5 C-Atomen, Anilin, Phenylhydrazin, N,N-Dimethylhydrazin und Hydrazinhydrat.

Bei Normalbedingungen feste $NH_2$-Gruppen enthaltende Verbindungen führt man der Destillationskolonne vorteilhafterweise gelöst in einem inerten Lösungsmittel zu. Flüssige Mono- oder Diamine, Amide oder Derivate des Hydrazins werden im allgemeinen unverdünnt in die Kolonne eingegeben.

Als Lösungsmittel, di für die als Feststoffe vorliegenden Verbindungen mit $NH_2$-Gruppen verwendet werden können, sind höherseidende Ester, chlorierte Kohlenwasserstoffe, Aromaten, Äther, Wasser und Phosphor enthaltende Verbindungen geeignet. Besonders geeignet sind Benzol, Toluol, Äthylbenzol und Xylole. Ganz besonders vorteilhaft ist es, Dioxan oder andere cyclische Äther, oder Phosphorsäureester, wie Tributylphosphat, zu verwenden. Im allgemeinen wird man bei der Festlegung des Systems Zu-reinigendes-Epoxid/$NH_2$-Gruppe enthaltende Verbindung-Lösungsmittel neben den Lösungseigenschaften für die Verbindung, die die Aminogruppe enthält, auch die Unterschiede der Siedepunkte von Epoxid und Lösungsmittel berücksichtigen. Für das erfindungsgemäße Verfahren ist es günstig, ein Lösungsmittel zu wählen, das mindestens 20°C höher siedet als das Epoxid. Lösungsmittel, die mindestens 50°C höher sieden als das Epoxid, eignen sich im allgemeinen gut. Vorteilhafterweise wählt man ein Lösungsmittel, das zwischen 60 und 110°C höher als das Epoxid siedet.

Die Konzentration an der die $NH_2$-Gruppe enthaltenden Verbindung in der zum Einsatz in die Destillationskolonne gelangenden Lösung kann in weiten Grenzen, je nach Art des Lösungsmittels, variiert werden. Gehalte von 0,1 bis 90 Gew.-% eignen sich im allgemeinen gut. Vorteilhafterweise betragen diese Konzentrationen 20 bis 80 Gew.-%, ganz besonders bevorzugt 30 bis 70 Gew.-%.

Die Menge an der die Aminogruppe enthaltenden Verbindung, die für das Verfahren gemäß der Erfindung im allgemeinen zur Anwendung gelangt, ist von dem Gehalt an Carbonylverbindungen in dem zu reinigenden Epoxid abhängig. Sie kann jedoch in weiten Grenzen schwanken. Ein Molverhältnis von der die $NH_2$-Gruppe enthaltenden Verbindung zu Carbonylverbindungen von 0,5:1 ist in den meisten Fällen bereits ausreichend. Vorteilhafterweise wählt man einen Bereich dieses Molquotienten von 1 bis 5:1. Ganz besonders vorteilhaft ist es, ein Molverhältnis von der die $NH_2$-Gruppe enthaltenden Verbindung zu Carbonylverbindungen von 1 bis 3:1 einzustellen.

Der Druck in der gemäß dem erfindungsgemäßen Verfahren verwendeten Destillationseinheit kann beispielsweise 0,01 bis 2,5 bar betragen. Der Druck wird zweckmäßigerweise so gewählt, daß das im oberen Teil der Kolonne anfallende gereinigte Epoxid mit Kühlwasser von beispielsweise 25°C kondensiert werden kann. Durch die Wahl des Druckes in der Kolonne sind auch die Druckverhältnisse in dem Teil der Destillationseinheit festgelegt, der sich zwischen den Zulaufstellen von Epoxid und Aminoverbindungen bzw. der Lösung von der die $NH_2$-Gruppe enthaltenden Verbindungen befindet. Gleichzeitig sind damit aber auch, da der Siedepunkt des Epoxids bei vorgegebenem Druck festliegt, die in deisem Kolonnenteil vorliegenden Temperaturen in etwa bestimmt. Je nach dem, welches Epoxid gereinigt werden soll, d.h. welche Temperatur am Kopf der Kolonne sich bei 1 bar einstellen würde, wählt man nun den Druck der Kolonne so, daß sich in dem Kolonnenstück, das durch die Zulaufstellen von Epoxid und Aminoverbindung bzw. der Lösung der die $NH_2$-Gruppe enthaltenden Verbindung begrenzt ist und das bevorzugt 1 bis 10 theoretische Trennstufen umfaßt, Temperaturen zwischen 10 und 150°C, vorzugsweise 20 bis 100°C, ganz besonders bevorzugt 30 bis 80°C, einstellen. Umfaßt der durch die Zulaufstellen begrenzte Teil der Kolonne mehr als eine theoretische Trennstufe, beispielsweise 5 Stufen, so wird sich im allgemeinen eine Temperaturdifferenz zwischen der untersten und der obersten Stufe einstellen. Die hierbei in diesem Teil der Kolonne festgestellten Extremtemperaturen, also die Höchstund die Tiefsttemperatur, sollen in den oben angegebenen Temperaturbereichen liegen.

Das Rücklaufverhältnis, unter dem die Destillationseinheit des erfindungsgemäßen Verfahrens betrieben wird, kann beispeilsweise 0,1 bis 10:1 betragen, wobei unter diesem Verhältnis das Mengenverhältnis von Rücklauf auf die Kolonne zu am Kopf entnommenem Destillat zu verstehen ist.

Das angegebene Rücklaufverhältnis kann über- oder unterschritten werden. Man kann beispielsweise auch bei wesentlich höheren Rücklaufverhältnissen arbeiten.

Das als Kopfprodukt oder in einem Seitenstrom nach dem erfindungsgemäßen Verfahren gewonnene gereinigte Epoxid enthält weniger als 100 ppm Carbonylverbindungen, gerechnet als Acetaldehyd. Vorzugsweise ist dieser Gehalt weniger als 50 ppm, besonders bevorzugt weniger als 20 ppm.

Neben der Gewinnung von gereinigtem Epoxid ist es auch möglich, sofern das in die Destillationseinheit eingespeiste Epoxid in Form Eines Gemisches vorliegt, die als Lösungsmittel fungierende Verbindung ganz oder teilweise zusammen mit dem Epoxid als Kopfprodukt zu isolieren. Auch in diesem Fall weisen die dann erhaltenen Gemische aus gereinigtem Epoxid und Lösungsmittel die vorgenannten Spezifikationen auf.

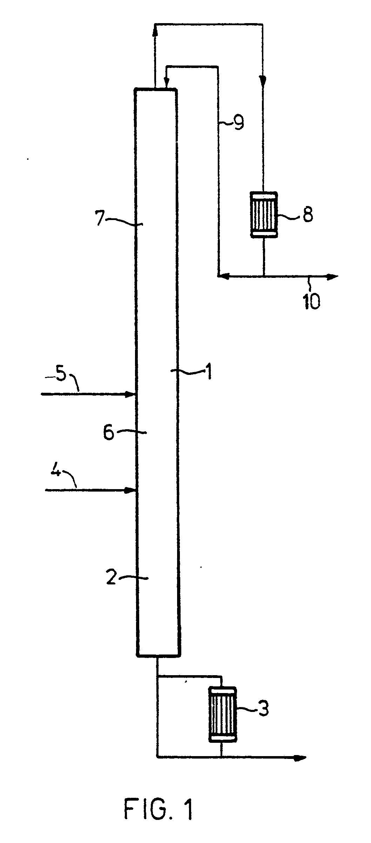

Eine bevorzugte Durchführungsform des erfindungsgemäßen Verfahrens wird im folgenden beispielhaft für die Reinigung von Propylenoxid beschrieben (siehe hierzu auch Fig. 1).

Man verwendet eine Destillationseinheit (1), deren Abtriebsteil (2), d.h. der unterhalb (4) befindliche Kolonnenabschnitt, 5 bis 8 theoretische Trennstufen umfaßt. Die Beheizung der Kolonne erfolgt mit einem Umlaufverdampfer (3). Der zwischen dem Einlauf einer Propylenoxid enthaltenden Lösung in Äthylbenzol (4) und dem Zulauf der n-Butylamin enthaltenden Lösung (5) liegende Kolonnenteil (6) umfaßt 3 bis 6 theoretische Trennstufen, die durch etwa 5 bis 8 praktische, als Glockenböden ausgebildete Stufen verwirklicht werden. Der oberhalb der Zulaufstelle (5) der n-Butylaminlösung befindliche Teil der Destillationseinheit (7) besteht aus 15 bis 20 theoretischen Trennstufen und ist als Füllkörperschuß mit Glasraschigringen ausgebildet. Das über Kopf gehende Propylenoxid wird in den Wärmeaustauscher (8) kondensiert. Der Rücklauf auf die Kolonne erfolgt über Leitung (9). Die Destillationseinheit (1) wird mit einer 10 bis 25 Gew.-% Propylenoxid enthaltenden Lösung in Äthylbenzol durch Leitung (4) beschickt. Neben Propylenoxid und Äthylbenzol sind in diesem Einsatzstrom (4) noch 0,5 bis 1,5 Gew.-% Acetaldehyd, 0,1 bis 0,8 Gew.-% Propionaldehyd, 0.05 bis 0,25 Gew.-% Methylformiat, 0,01 bis 0,2 Gew.-% Acrolein, etwa 1 Gew.-% Wasser und Spuren von Aceton enthalten. Über Leitung (5) wird der Kolonne eine Lösung zugeführt, die 40 bis 50 Gew.-% n-Butylamin und 20 bis 30 Gew.-% Wasser in Methanphosphonsäuredimethylester enthält. Das Molverhältnis von n-Butylamin zu Carbonylverbindungen beträgt 1,1 bis 2,5:1. Die Destillationseinheit wird bei einem Druck von 1,0 bis 1,5 bar betrieben. Das Rücklaufverhältnis liegt bei 5 bis 8:1. In dem mit (6) bezeichneten Teil der Kolonne beträgt die Temperatur 50 bis 70°C. Am Kopf der Kolonne fällt gereinigtes Propylenoxid an, das nach Kondensation über Leitung (10) ausgetragen wird. Dieses Produkt enthält weniger also 50 ppm Carbonylverbindungen. Der Wassergehalt liegt unter 100 ppm. Stickstoff enthaltende Verbindungen sind nicht nachweisbar.

Die Vorteile des erfindungsgemäßen Verfahrens lassen sich wie folgt zusammenfassen:

1) Hohe Reinheit des gewonnenen Epoxids;
2) Geringer technischer Aufwand durch einfache Verfahrensmaßnahmen, die man als neuartige Gegenstrom-Reaktivdestillation bezeichnen kann;
3) Vernachlässigbarer Verlust an Epoxid;
4) Entfernung sämtlicher störender Carbonylverbindungen in einer Destillationskolonne bei gleichzeitig bestehender Möglichkeit, das Epoxid in der selben Kolonne von einem Lösungsmittel abzutrennen;
5) Anwendbarkeit des Verfahrens auch für Fälle, wo eine Carbonylgruppen enthaltende, das Epoxid verunreinigende Verbindung aus einer anderen Verbindung durch die thermische Belastung während der Destillation in Freiheit gesetzt wird.
6) Gewinnung von reinem Epoxid unter Abtrennung von Lösungsmittel und von den Carbonylverbindungen in einer einzigen Destillationskolonne (gegenüber mindestens zwei Destillationskolonnen, wie sie z.B. nach dem Verfahren gemäß DT—OS 2 454 115 zur Erzeilung ähnlicher Ergebnisse erforderlich sind).

### Beispiele
(s. auch Fig. 1)

### Beispiel 1
Eine ausschließlich als Glockenbodenkolonne ausgestaltete Destillationseinheit von 5 cm Durchmesser, deren Abtriebsteil 10 praktische Böden umfaßt und deren Verstärkerteil insgesamt aus 25 praktischen Böden besteht, wird stündlich mit 250 ml 98,5%igem Vinyloxiran (Dichte: 0,87), das 1,2 Gew.-% Carbonylverbindungen und 0,3 Gew.-% Butendiole und Butendiolmonoacetate sowie geringe Mengen an Wasser enthält, über Leitung (4) beaufschlagt. Dieser Produktstrom wird vor Einlauf in die Kolonne auf 65°C vorgewärmt. Die Kolonne ist mit einem Umlaufverdampfer und am Kopf mit einem Kühler und einem Destillatbehälter ausgestattet. Die 1,2 Gew.-% Carbonylverbindungen bestehen im einzelnen aus folgenden Verbindungen: 0,5 Gew.-% Crotonaldehyd, 0,1 Gew.-% Acetaldehyd, 0,25 Gew.-% Methyläthylketon, 0,08 Gew.-% Propionaldehyd und 0,27 Gew.-% Acrolein. Fünf praktische Böden oberhalb des Einlaufes (4) des Vinyloxirans in die Kolonne, also insgesamt auf Höhe des 15. praktischen Bodens, vom Sumpf aus gerechnet, führt man über Leitung (5) eine 35 gew.-%ige Lösung von Semicarbazid in Dioxan in einer Menge von 13,4 g pro Stunde in die Kolonne ein. Der Molquotient von Semicarbazid zu Carbonylverbindungen beträgt somit also 1,5:1.

Die Kolonne wird bei einem Kopfdruck von 1 bar betrieben. Das Rücklaufverhältnis beträgt 5:1, sodaß in der Stunde etwa 1,23 1 Vinyloxiran über (9) wieder auf die Kolonne gegeben werden. Die Kopftemperatur stellt sich bei 68° bis 69°C ein. Die Temperatur zwischen dem 13. und 14. Boden beträgt etwa 70 bis 72°C.

Aus dem Destillatbehälter entnimmt man über (10) stündlich 214 g Vinyloxiran, das eine Reinheit von 99,95% besitz. Der Gehalt an Carbonylverbindungen liegt unter 50 ppm. Der Vergleich der Mengenströme (4) und (10) zeigt, daß der Verlust von Vinyloxiran lediglich 0,5% beträgt, was praktisch innerhalb der Meßgenauigkeit liegt.

### Beispiel 2
Man verwendet eine Destillationskolonne, die über die gesamte Länge einen Durchmesser von 5 cm besitzt. Der Abtriebsteil besteht aus einem mit 4 × 4 mm Glasraschigringen gefüllten Glasschuß von 50 cm Länge, während der Verstärkerteil aus einem von 4 Glockenböden enthaltendem Rohr und einem weiteren Rohr, das mit Edelstahl-Maschendrahtringen gefüllt ist, zusammengesetzt ist. Der Glockenbodenschuß befindet sich unmittelbar oberhalb der Einlaufstelle des Produktes. Auf diesen Schuß folgt nach oben hin der mit den Maschendrahtringen gefüllte Schuß, der eine Länge von 1,5 m besitzt. Die Kolonne wird mit einem Fallfilmverdampfer beheizt. In die bei einem Druck von 0,015 bar betriebene Kolonne (Kopfdruck) werden ständlich 450 ml Styroloxid (Dichte: 1,06), das vor Eintritt in die Kolonne auf 70°C vorgewärmt wird, eingegeben. Das Styroloxid besitzt eine Reinheit von 99,2 Gew.-%. Die Verunreinigungen bestehen im wesentlichen aus Aceton, Acetaldehyd und Methyläthylketon, wobei Aceton mit 0,6 Gew.-% den Hauptanteil der Carbonylgruppen enthaltenden Verunreinigungen darstellt. Neben diesen Verunreingungen enthält das Styroloxid noch 0,12 Gew.% Wasser.

Die Einlaufstelle einer 25 gew.-%igen Lösung von Acethydrazid in Tributylphosphat befindet sich am oberen Ende des vier Glockenböden enthaltenden Kolonnenteils. Stündlich werden von dieser Lösung 25 g in die Kolonne eingegebenen.

Das Rücklaufverhältnis beträgt 1:1 und die Temperatur am Kopf der Kolonne liegt bei 71—72°C. Als Kopfprodukt gewinnt man in der Stunde 470 g Styroloxid, dessen Gehalt an Carbonylverbindungen unter 100 ppm liegt. Ein Verlust an Styroloxid ist nicht feststellbar.

Beispiel 3

Ein Produktstrom, der neben Benzol 32,5 Gew.-% Propylenoxid, 0,4 Gew.-% Propylenglykol, 0,1 Gew.-% Acetaldehyd, 0,05 Gew.-% Methylformiat und 0,01 Gew.-% Aceton sowie 0,3 Gew.-% Wasser enthält, wird in einer Menge von 500 ml pro Stunde in eine Destillationskolonne eingegeben, die insgesamt mit 45 Glockenböden ausgestattet ist. Die Beheizung der Kolonne, die einen Durchmesser von 5 cm besitzt, erfolgt mit einem Umlaufverdampfer.

Der Einlauf des Propylenoxid enthaltenden Stromes in die Kolonne befindet sich zwischen dem 15. und 16. Boden, vom Sumpf aus gerechnet. Zwischen dem 25. und 26. Boden erfolgt die Eingabe von stündlich 0,92 g einer 64 gew.%igen wäßrigen Lösung von Hydrazin. Somit ergibt sich ein Molverhältnis von Carbonylverbindungen zu Hydrazin von 1,3:1.

Die Kolonne betriebt man bei einem Kopfdruck von 1,33 bar. Die Kopftemperatur beträgt 43—44°C, das Rücklaufverhältnis liegt bei 7:1. Die Kondensation der Kopfdämpfe wird mit Kühlwasser vorgenommen.

Als Destillat wird der Kolonne stündlich in einer Menge von 169 ml Propylenoxid entnommen. Das gewonnene Produkt enthält weniger als 10 ppm Acetaldehyd und weniger als 200 ppm Wasser. Methylformiat und Aceton sind nicht mehr nachweisbar.

Als Sumpfprodukt werden pro Stunde 290 g Benzol ausgetragen. Das Sumpfprodukt enthält weniger als 0,01 Gew.-% Propylenoxid.

Beispiel 4

Ein Gemisch, das neben Äthylbenzol 45,2 Gew.-% Propylenoxid, 0,08 Gew.-% Acetaldehyd, 0,01 Gew.-% Methylformiat, 0,05 Gew.% Propionaldehyd sowie 0,5 Gew.-% Wasser und geringe Mengen an Propylenglykol enthält, wird mit einer Menge von 450 g pro Stunde in eine mit 50 Glockenböden versehene Destillationskolonne von 5 cm Durchmesser eingegeben. Der Produktzulauf, d.h. die Eingabe der Lösung von Propylenoxid in Äthylbenzol, in die mit einem Fallfilmverdampfer beheizte Kolonne liegt in Höhe des 20. Bodens, vom Sumpf ausgerechnet. Zwischen dem 30. und 31. Boden, ebenfalls vom Sumpf her gezählt, werden der Kolonne stündlich 17 g einer 10 Gew.-%igen Lösung von n-Butylamin in Dioxan zugeführt. Das molare Verhältnis von Amin zu Carbonylverbindungen beträgt 1,8:1. Die Kolonne wird bei Normaldruck betrieben. Bei einer Kopftemperatur von 34°C und einem Rücklaufverhältnis von etwa 6,5:1 fallen stündlich 244 ml Propylenoxid als Destillat an. Dieses Produkt enthält nur noch 10—15 ppm Acetaldehyd. Der Gehalt an anderen Carbonylverbindungen liegt unter 50 ppm.

Beispiel 5

Das zu reinigende 2,3-trans-Butylenoxid liegt als Lösung in tert.-Butanol vor. Der Gehalt an Epoxid beträgt 70,2 Gew.-%. Weiterhin sind in der Lösung 0,15 Gew.-% Methyläthylketon, 0,1 Gew.-% n-Butyraldehyd und 0,08 Gew.-% Propionaldehyd sowie Spuren von Acrolein und Wasser vorhanden.

Als Destillationskolonne wird eine Füllkörperkolonne von 2,5 m Länge und 4,5 cm Durchmesser verwendet, die bis auf ein 5 Glockenböden enthaltendes Mittelstück mit 4 × 4 mm Glasringen gefüllt ist. Der Einlauf des auf 55°C vorgewärmten, das 2,3-trans-Butylenoxid enthaltenden Produktstromes liegt unterhalb des Glockenbodenschusses, während die Eingabe einer 35 gew.-%igen Lösung von Phenylhydrazin in Dioxan an dessen oberen Ende noch vor Beginn des oberen Füllkörperschusses erfolgt.

Die das Epoxid enthaltende Lösung wird in einer Menge von 350 ml pro Stunde und die Lösung von Phenylhydrazin in Dioxan in einer Menge von 9,5 g pro Stunde in die Kolonne eingegeben, so daß das Molverhältnis von Carbonylverbindungen zu Phenylhydrazin 2,2:1 beträgt. Die Kolonne wird unter Normaldruck betrieben. Das Rücklaufverhältnis beträgt 5:1. Bei einer Kopftemperatur von 56—57°C erhält man als Destillat in der Stunde 237 ml 2,3-trans-Butylenoxid, das weniger als 50 ppm Carbonylverbindungen enthält. Als Sumpfprodukt wird das tert.-Butanol aus der Kolonne abgezogen.

Beispiel 6

Das zu reinigende Epoxid ist wiederum 2,3-trans-Butylenoxid. Die verwendete Destillationskolonne besitzt die in Beispiel 5 angegebenen Abmessungen. Die Eingabe des Produkt enthaltenden Stromes erfolgt ebenfalls wieder unterhalb des die Glockenböden enthaltenden Kolonnenschusses. Das abzutrennende Butylenoxid liegt im benzolischer Lösung vor, die die folgende Zusammensetzung besitzt. 23,3 Gew.-% 2,3-trans-Butenoxid, 0,15 Gew.-% n-Butyraldehyd, 0,12 Gew.-% Methyläthylketon sowie 0,2 Gew.-% Diacetyl und geringe Mengen Wasser.

Diese Lösung wird in einer Menge von 300 g pro Stunde in die Kolonne eingefahren. Am oberen Ende des die Glockenböden enthaltenden Teils der Kolonne gibt man stündlich 1,7 ml Äthylendiamin in die Kolonne ein.

Die Kolonne wird unter Normaldruck betrieben. Bei einer Temperatur am Kopf von 57°C und einem Rücklaufverhältnis von 9:1 erhält man pro Stunde 68,5 g eines 2,3-butylenoxids als Destillat, das weniger als 100 ppm Carbonylverbindungen enthält.

**Patentansprüche**

1. Verfahren zur Reinigung von Epoxiden, dadurch gekennzeichnet, daß man das Epoxid oder ein Epoxid enthalten des Gemisch, das, bezogen auf das Epoxid, bis zu 2 Gew.-% 1 bis 5 C-Atome enthaltende Carbonylverbindungen enthält, in den mittleren Bereich einer Destillationseinheit einleitet, oberhalb des Zulaufs des Epoxids enthaltenden Produktstromes eine Verbindung, die ein oder mehrere unsubstituierte NH$_2$-Gruppen aufweist, einleitet und oberhalb der Einleitungsstelle dieser mindestens eine NH$_2$-Gruppe enthaltenden Verbindung in die Destillationskolonne das gereinigte Epoxid als Kopfprodukt oder in einem Seitenstrom abzieht.

2. Verfahren nach Anspruch 1, dadurch gekennzeichnet, daß man eine Destillationseinheit verwendet, die einen Abtriebsteil mit 1 bis 50 theoretischen Trennstufen, einen Aufkonzentrierteil mit 1 bis 70 theoretischen Trennstufen, eine Verdampfereinheit und eine Kondensationseinrichtung enthält, wobei man zwischen dem Abtriebsteil und dem Aufkonzentrierteil das Epoxid oder das Epoxid enthaltende Gemisch, und 1 bis 20 theoretische Trenntstufen oberhalb davon mit mindestens eine freie NH$_2$-Gruppe enthaltende Verbindung einleitet und 1 bis 50 theoretische Trennstufen oberhalb der Einleitungsstelle das gereinigte Epoxid als Kopfprodukt oder in einem Seitenstrom abzieht.

3. Verfahren nach Anspruch 1 und 2, dadurch gekennzeichnet, daß man Äthylenoxid, Propylenoxid, 1,2-Epoxibutan, 2,3-Epoxibutan, Vinyloxiran oder Styroloxid oder ein Gemisch das eines dieser Epoxide enthält, wobei das Epoxid oder das Epoxid enthaltende Gemisch, bezogen auf das Epoxid, bis zu 2 Gew.-% 1 bis 5 C-Atome enthaltende Carbonylverbindungen enthält, einer Destillationseinheit zuführt, die aus einem Abtriebsteil mit 1 bis 20 theoretischen Trennstufen, einem Aufkonzentrierteil, einer Verdampfereinheit und einer Kondensationsvorrichtung besteht, wobei man 1 bis 10 theoretische Trennstufen oberhalb des Zulaufs des Epoxids oder des Epoxid enthaltenden Gemisches eine Verbindung mit einer unsubstituierten NH$_2$-Gruppe einleitet und bei einem Kopfdruck von 0,01 bis 2,5 bar und einem verhältnis von Rücklauf auf die Kolonne zu am Kopf entnommenem Distillat von 0,1 bis 10:1 das gereinigte Epoxid 5 bis 40 theoretische Trennstufen oberhalb der Einleitungsstelle für die eine NH$_2$-Gruppe aufweisende Verbindung, als Kopfprodukt oder in einem Seitenstrom abzieht.

4. Verfahren nach Anspruch 3, dadurch gekennzeichnet, daß man als Epoxid Propylenoxid oder Gemisch einsetzt, die Propylenoxid enthalten.

5. Verfahren nach Anspruch 1 bis 4, dadurch gekennzeichnet, daß der Kolonnenteil zwischen den beiden Zulaufstellen als Bodenkolonne ausgeführt ist.

6. Verfahren nach Anspruch 1 bis 5, dadurch gekennzeichnet, daß man ein Epoxid enthaltendes Gemisch einleitet, das Äthylbenzol, Methylphenylcarbinol, Toluol, Xylol, Benzol, Essigsäure, tert.Butylalkohol, Dichlorpropan oder Wasser enthält.

7. Verfahren nach Anspruch 1 bis 6, dadurch gekennzeichnet, daß man als Verbindung mit einer unsubstituierten NH$_2$-Gruppe ein Monoamin der Formel R—NH$_2$ (wobei R ein substituierter oder unsubstituierter geradkettig oder verzweigt vorliegender aliphatischer oder ein aromatischer Rest ist), oder ein C$_1$- bis C$_3$-Alkylendiamin oder ein Phenylendiamin oder ein Aminal, oder ein Säureamid einer Carbon-, Sulfon- oder Phosphonsäure, oder Harnstoff oder einen N-Alkyl oder N,N-Dialkylharnstoff, oder eine Aminocarbonsäure oder Anthranilsäure, oder Cyanamid oder Hydroxylamin einsetzt.

8. Verfahren nach Anspruch 1 bis 7, dadurch gekennzeichnet, daß man die eine NH$_2$-Gruppe enthaltende Verbindung in Form einer 30 bis 70 Gew.-%-igen Lösung einsetzt.

9. Verfahren nach Anspruch 1 bis 8, dadurch gekennzeichnet, daß man Hydrazin oder ein Hydrazinderivat in einer Menge einsetzt, daß der Molquotient von Hydrazin oder Hydrazinderivat zu Carbonylverbindung 1 bis 3:1 beträgt.

10. Verfahren nach Anspruch 1 bis 9, dadurch gekennzeichnet, daß das abgezogene Epoxid weniger als 100 ppm Carbonylverbindungen, gerechnet als Acetaldehyd, enthält.

**Revendications**

1. Procédé de purification d'époxydes, caractérisé en ce qu'on fait arriver l'époxyde ou un mélange contenant l'époxyde qui, par rapport à ce dernier, renferme jusqu'à 2% en poids de composés carbonyliques ayant 1 à 5 atomes de carbone, dans la région moyenne d'un appareil de distillation, on fait arriver au-dessus de l'admission du courant de produit renfermant l'époxyde un composé qui porte un ou plusieurs groupes NH₂ non substitués et on décharge au-dessus du point d'introduction, dans la colonne de distillation de ce composé portant au moins un groupe NH₂, l'époxyde purifié comme produit de tête ou en un courant latéral.

2. Procédé suivant la revendication 1, caractérisé en ce qu'on utilise un appareil de distillation qui comprend une partie de séparation comprenant 1 à 50 étages théoriques, une partie de concentration comprenant 1 à 70 étages théoriques, un dispositif d'évaporation et un dispositif de condensation, et on fait arriver l'époxyde ou le mélange contenant l'époxyde entre la partie de séparation et la partie de concentration et 1 à 20 étages théoriques au-dessus, le composé portant au moins un groupe NH₂ libre, et on décharge, 1 à 50 plateaux théoriques au-dessus du point d'introduction, l'époxyde purifié comme produit de tête ou en un courant latéral.

3. Procédé suivant la revendication 1 ou 2, caractérisé en ce qu'on fait arriver de l'oxyde d'éthylène, de l'oxyde de propylène, du 1,2-époxybutane, du 2,3-époxybutane, du vinyloxirane ou de l'oxyde de styrène ou un mélange qui contient l'un de ces époxydes, l'éoxyde ou le mélange qui le contient renfermant, par rapport à l'époxyde, jusqu'à 2% en poids de composés carbonyliques renfermant 1 à 5 atomes de carbone, à un appareil de distillation qui est composé d'une partie de séparation ayant 1 à 20 étages théoriques, d'une partie de concentration, d'un dispositif d'évaporation et d'un dispositif de condensation, en faisant arriver, 1 à 10 étages théoriques au-dessus de l'admission de l'époxyde ou du mélange contenant l'époxyde, un composé porteur d'un groupe NH₂ non substitué et en déchargeant, à une pression de tête de 0,01 à 2,5 bars (1 à 250 kPa) et à un rapport du reflux dans la colonne au distillat prélevé en tête de 0,1 à 10:1, l'époxyde purifié 5 à 40 étages théoriques au-dessus du point d'introduction du composé porteur d'un groupe NH₂, comme produit de tête ou en un courant latéral.

4. Procédé suivant la revendication 3, caractérisé en ce qu'on utilise comme époxyde l'oxyde ·de propylène ou des mélanges qui referment de l'oxyde de propylène.

5. Procédé suivant l'une des revendications 1 à 4, caractérisé en ce qui la partie de la colonne se trouvant entre les deux points d'admission est réalisée comme colonne à plateaux.

6. Procédé suivant l'une des revendications 1 à 5, caractérisé en ce qu'on introduit un mélange contenant un époxyde qui renferme de l'éthylbenzene, du méthylphénylcarbinol, du toluène, du xylène, du benzène, de l'acide acétique, de l'alcool tertio-butylique, du dichloropropane ou de l'eau.

7. Procédé suivant l'une des revendications 1 à 6, caractérisé en ce qu'on utilise comme composé porteur d'un groupe NH₂ non substitué une mono-amine de formule R—NH₂ (dans laquelle R est un reste aliphatique substitué ou non substitué se présentant en chaîne droite ou en chaîne ramifiée ou un reste aromatique) ou une alkylène-diamine en C₁ à C₃ ou une phénylène-diamine ou un aminal ou un amide d'un acide carboxylique, sulfonique ou phosphonique ou l'urée ou une N-alkyl- ou N,N-dialkyl-urée ou un acide aminocarboxylique ou l'acide anthranilique ou un cyanamide ou l'hydroxylamine.

8. Procédé suivant l'une des revendications 1 à 7, caractérisé en ce qu'on utilise un composé porteur d'un groupe NH₂ sous la forme d'une solution à 30—70% en poids.

9. Procédé suivant l'une des revendications 1 à 8, caractérisé en ce qu'on utilise l'hydrazine ou un dérivé d'hydrazine en une quantité choisie de manière que le rapport molaire de l'hydrazine ou du dérivé d'hydrazine au composé carbonylique ait une valeur de 1 à 3:1.

10. Procédé suivant l'une des revendications 1 à 9, caractérisé en ce que l'époxyde déchargé renferme moins de 100 parties par million de composés carbonyliques, exprimés en acétaldéhyde.

## Claims

1. Process for the purification of epoxides, characterised in that the epoxide, or an epoxide-containing mixture, which contains, relative to the epoxide, up to 2% by weight of carbonyl compounds containing 1 to 5 C atoms, is passed into the middle region of a distillation unit, a compound which contains one or more unsubstituted NH₂ groups is passed in above the inlet of the epoxide-containing product stream and the purified epoxide is taken off as the top product, or in a side stream, above the point at which this compound containing at least one NH₂ group enters the distillation column.

2. Process according to Claim 1, characterised in that a distillation unit is used which contains a stripping section with 1 to 50 theoretical plates, a concentrating section with 1 to 70 theoretical plates, a vaporiser unit and a condensing device, the epoxide or the epoxide-containing mixture being passed in between the stripping section and the concentrating section, the compouind containing at least one free NH₂ group being passed in 1 to 20 theoretical plates above this point and the purified epoxide being taken off as the top product, or in a side stream, 1 to 50 theoretical plates above the feed point.

3 Process according to Claim 1 and 2, characterised in that ethylene oxide, propylene oxide, 1,2-epoxybutane, 2,3-epoxybutane, vinyloxirane or styrene oxide or a mixture which contains one of these epoxides, the epoxide or the epoxide-containing mixture containing, relative to the epoxide, up to 2% by weight of carbonyl compounds containing 1 to 5 C atoms, is fed into a distillation unit which consists of a stripping section with 1 to 20 theoretical plates,a concentrating section, a vaporiser unit and a condensing device, a compound with an unsubstituted $NH_2$ group being passed in 1 to 10 theoretical plates above the inlet for the epoxide or the epoxide-containing mixture and the purified epoxide being taken off as the top product, or in a side stream, 5 to 40 theoretical plates above the inlet for the compound containing an $NH_2$ group, under an overhead pressure of 0.01 to 2.5 bars and at a ratio of reflux onto the column to distillate removed at the top of 0.1 to 10:1.

4. Process according to Claim 3, characterised in that propylene oxide or mixtures containing propylene oxide are employed as the epoxide.

5. Process according to Claim 1 to 4, characterised in that the column section between the two feed points is in the form of a tray column.

6. Process according to Claim 1 to 5, characterised in that an epoxide-containing mixture which contains ethylbenzene, methylphenylcarbinol, toluene, xylene, benzene, acetic acid, tert.-butyl alcohol, dichloropropane or water is passed in.

7. Process according to Claim 1 to 6, characterised in that a monoamine of the formula $R—NH_2$ (R being a substituted or unsubstituted straight-chain or branched aliphatic radical or an aromatic radical), or a $C_1$- to $C_3$-alkylenediamine or a phenylenediamine or an aminal, or an acid amide of a carboxylic acid, sulphonic acid or phosphonic acid, or urea or an N-alkyl or N,N-dialkyl-urea, or an aminocarboxylic acid or anthranilic acid, or cyanamide or hydroxylamine is employed as the compound with an unsubstituted $NH_2$ group.

8. Process according to Claim 1 to 7, characterized in that the compound containing an $NH_2$ group is employed in the form of a 30 to 70% strength by weight solution.

9. Process according to Claim 1 to 8, characterised in that the hydrazine or the hydrazine derivative is employed in an amount that the molar quotient of hydrazine or hydrazine derivative to carbonyl compound is 1 to 3:1.

10. Process according to Claim 1 to 9, characterised in that the epoxide removed contains less than 100 ppm of carbonyl compounds, calculated as acetylaldehyde.

FIG. 1